# EUROPEAN PATENT SPECIFICATION

(11) **EP 4 563 193 B1**
(45) Date of publication and mention of the grant of the patent: **09.09.2026**
(21) Application number: 23220413.1
(22) Date of filing: 27.12.2023
(51) Int. Cl.: A61N 5/10, G01T 1/20

(54) **PHANTOM DEVICE FOR TELERADIOTHERAPY TO DETERMINE THE SPATIAL DISTRIBUTION OF DOSE OF IONIZING RADIATION**
PHANTOMGERÄT FÜR DIE TELERADIOTHERAPIE UM DIE RÄUMLICHE VERTEILUNG DER DOSIS IONISIERENDER STRAHLUNG ZU BESTIMMEN
APPAREIL FANTÔME POUR TÉLÉRADIOTHÉRAPIE POUR DÉTERMINER LA DISTRIBUTION SPATIALE DE LA DOSE DE RAYONNEMENT IONISANT

(30) Priority: 30.11.2023 PL 44694423
(43) Date of publication of application: 04.06.2025
(73) Proprietor: Politechnika Krakowska im. Tadeusza Kosciuszki, 31-115 Kraków (PL)
(72) Inventor: BARAN, Mateusz, 31-408 Krakow (PL); JELEN-STOLARSKA, Kinga, 43-310 Bielsko-Biala (PL); NABAGLO, Tomasz, 31-572 Krakow (PL); RACHWAL, Bartlomiej, 30-653 Krakow (PL); RZECKI, Krzysztof, 30-498 Krakow (PL); TABOR, Zbislaw, 32-020 Wieliczka (PL); WALIGORSKI, Michal, 30-117 Krakow (PL)
(74) Representative: Kacperski, Andrzej

(56) References cited:
- US-A1- 2009 236 510
- US-A1- 2013 006 035
- US-A1- 2014 217 295
- LACROIX FRÃ DERIC ET AL: "Clinical prototype of a plastic water-equivalent scintillating fiber dosimeter array for QA applications", MEDICAL PHYSICS, AIP, MELVILLE, NY, US, vol. 35, no. 8, 17 July 2008 (2008-07-17), pages 3682 - 3690, XP012116197, ISSN: 0094-2405, DOI: 10.1118/1.2953564

## Description

The subject of the invention is a phantom device for teleradiotherapy, used to determine the spatial distribution of the dose of ionizing radiation realized within the active volume of the phantom, for comparison with the planned distribution of dose.

Real-time mapping of the spatial distribution of the ionizing radiation dose in the treated body volume of a patient undergoing oncological teleradiotherapy is important with regard to implementation of many aspects of quality assurance procedures in oncological radiotherapy, especially in dynamic procedures (e.g. Dynamic Conformal Arc Radiotherapy-DCAT or Intensity Modulated Arc Therapy-IMAT). Real-time assessment of such a distribution in terms of the difference between spatial distribution and local dose values of the dose delivered to the phantom and the distribution and values of dose planned, makes it possible to evaluate the quality of teleradiotherapy performed dynamically, enabling optimization of the individual radiotherapy plan for the patient prior to its delivery. The precision and accuracy of the procedure are also enhanced, particularly in terms of dynamic administration of the highest accepted dose to the treated volume while simultaneously decreasing the dose administered to volumes surrounding the tumour and to critical organs.

Known are phantoms incorporating passive detectors (e.g. TL or films) which are used in quality assurance procedures in radiotherapy and which reproduce to a degree the geometry of the patient's body.. Fixed measurement points are then determined in the active volume of such phantoms at which radiation detectors are positioned, without allowance to vary their positioning, where the dose distribution within the active volume is obtained by interpolating dose values between these measurement points. Measurement results are obtained only after reading the passive detectors, e.g. a few hours after the measurement has been made. Also, the dose distribution and dose values obtained by applying such phantoms are not precise enough.

To achieve the assumed goal of real-time mapping of the spatial distribution of radiation dose, single detectors, scintillation detector systems or optical fibers (fibers) made of scintillation material are also applied as detectors of ionizing radiation, whereby the location of the scintillation light flash is based on the difference in light intensity at the ends of the scintillation fiber, or by analyzing the distribution of scintillation signals read from many fibers placed within the active volume of the phantom.

US 2014217295A1 discloses a phantom device able to determine two- or three-dimensional (2D or 3D) dose distributions, based on the application of multiple optical fibers acting as scintillators over their lengths, three-dimensionally distributed within the active volume of the phantom or two-dimensionally located along a single plane, and a photo-sensitive device enabling two-dimensional digital readout of signals from the end faces of these scintillators attached to the surface of such a photo-sensitive device (e.g., a digital camera matrix). By numerically analyzing the 2D distribution obtained from the photo-sensitive device and knowing the positions of individual optical fibers within the active volume of the phantom, it is possible, using algorithms applied in tomographic reconstruction, to obtain in an approximate manner the values of dose and positions of points located along the optical fibers where scintillations due to ionization events occurred.

US 2021311212A1 discloses a phantom in the form of e.g. a cylindrical layer of appropriate thickness made of tissue-like material, covered on its inside with a layer of scintillation material, and a photo-sensitive device (e.g. a digital camera) placed along the axis of this cylindrical layer, configured to record the image of this scintillation layer from the interior of the phantom while the phantom is being irradiated by an X-ray beam of a medical accelerator beam from directions generally perpendicular to the axis of the phantom. By knowing the positions in space of points on the scintillator layer where the light signal has been generated due to ionizing events resulting from irradiation of the phantom by the medical accelerator beam and using algorithms applied in tomographic reconstruction, it is possible to reproduce the 3D distribution of dose in the air inside the phantom. Obtaining a 3D dose distribution inside the phantom in a medium other than air (water, tissue) requires additional recalculation of the measured distribution.

US 2012292517A1 describes a dosimetry system enabling real-time assessment of two-dimensional (2D) dose distribution. For this purpose, a system of single scintillation fibers is used in which the position of the scintillation flash along the fiber axis can be assessed in real time by measuring the difference in the intensity of light signals recorded at both ends of a given fiber. Parallel arrangement of several optical fibers combined with a fast electronic system enabling time synchronization of differential readout of signals from these individual fibers enables the 2D dose distribution for the active surface of the system to be reconstructed. US2022326402A1 discloses variations of megavoltage photon beam (MV) detectors that can be used to obtain high-resolution dynamic images and dose measurements in patients. One variation of such a detector consists of a fiber optic scintillation plate, a photodiode array configured to receive light data from the fiber optics, and readout electronics. In some embodiments, the scintillation fiber optic plate contains one or more optical fibers focused on a radiation source. The fiber diameters can be smaller than the pixels of the photodiode array. In some embodiments, the fiber diameter is on the order of about 2 to about 100 times smaller than the pixel width of the photodiode array.

US 2009/236510 A1 discloses a phantom comprising a plurality of slabs comprising scintillating optical fibers. Each of these fibers is coupled to a collection optical fiber. The signals from the collection optical fibers are sent to a photodetector.

The technical problem to be solved according to the invention concerns the possibility of real-time measurement of dose distribution at several points within the active volume of the phantom, while making it possible to simultaneously select these points or areas within the active volume without the need to reconfigure the phantom mechanically. The choice of particular areas within the active volume may be dictated by clinical considerations, e.g., by the location of critical or target areas for an individual plan, or the selection of particular dynamic radiotherapy procedures (e.g. Dynamic Conformal Arc Radiotherapy-DCAT or Intensity Modulated Arc Therapy-IMAT). The possibility to dynamically reconfigure selected points within the active area by selecting them in the phantom using a control program, as well as the ability to dynamically control the operation of the phantom, e.g. by reconfiguring it during the irradiation procedure, are expected.

The phantom device for teleradiotherapy purposes, as represented by this invention to assess the spatial distribution of the dose of ionizing radiation realized against the dose distribution planned, contains, within the active volume of this phantom device, many individual measurement points corresponding to voxels of this active volume, at which points the dose distribution is assessed, each point being equipped with an identical individual detector of ionizing radiation, each such detector being individually connected by a signal-conducting cable to a module which reads, records and processes this signal.

The essence of the solution according to the invention is that individual ionizing radiation detectors corresponding to the voxels of the active volume have the form of a sequence of identical regular solids, individually shielded around all faces by shielding and arranged to contact each other, placed within basic cassettes, whereby several layers of cassettes are located one above the other and are shifted by a regular step.

The basic cassettes arranged in layers as modules are arranged repetitively in space in a regular geometric arrangement, fitting the active measuring volume of the phantom device as closely as possible.

Each individual detector has its own independent, straight signal-conducting cable located along a symmetry axis of the detector in the form of a straight line, which does not collide with other detectors. The shielding of individual detectors is equipped in its walls with through channels for signal-conducting cables of other detectors located in adjacent layers.

**In** preferred versions, individual detectors may have the form of cubes, regular prisms with a regular hexagonal base, or a honeycomb arrangement of rhombic dodecahedra.

**In** the presented solution, the device contains 64 individual detectors in the form of cubes in 4 layer modules, 16 in each, shifted by a fixed step.

Cassettes with detectors in the form of cubes and regular prisms are closed with a lower cover and an upper cover, acting as shielding of individual detectors within each cassette .

The cassettes have an outline defining a puzzle-type fin connector. Layered cassette modules can be connected to subsequent cassette modules, increasing the active volume of the phantom device, ensuring that the active measuring volume of the phantom device expanded in this manner is fitted with detectors as closely as possible.

Individual ionizing radiation detectors are preferably scintillators, the shielding is light-tight, and the signal-conducting cables are optical fibers. Dose detectors in the form of scintillators are coupled to individual photomultipliers.

Signal-conducting cables leading from individual ionizing radiation detectors may be optical fibers or electric cables.

The advantage of the solution according to the invention is the ability to assess in real time the quality of the patient's individual treatment plan in radiation oncology, in terms of the ability to execute this treatment plan, but also considering the technical limitations of the radiotherapy device used (linear medical accelerator). The use of a reconfigurable phantom device allows one to increase the accuracy of the implementation of an individual treatment plan, compared to previous methods used in quality assurance procedures of treatment plans.

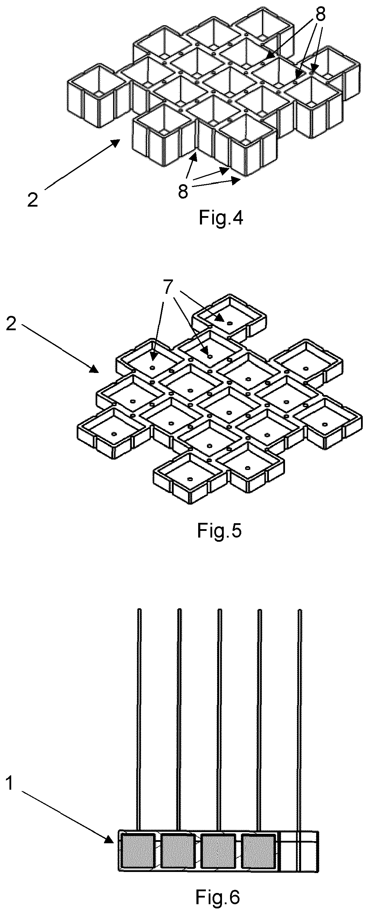

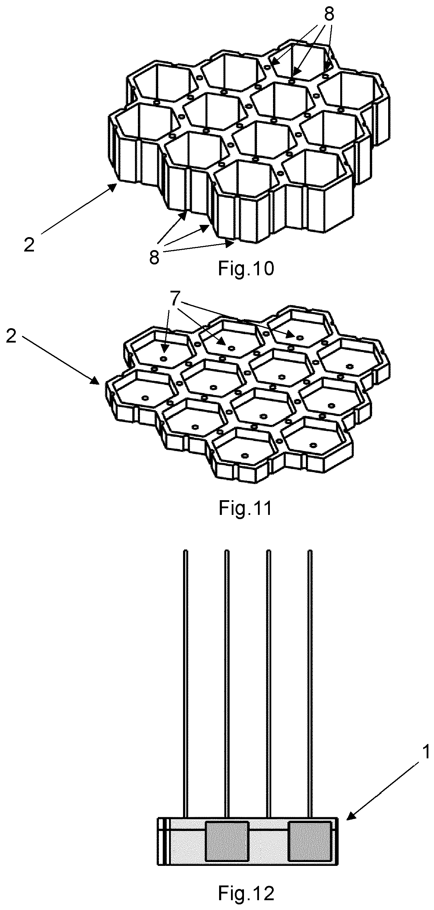

The solution according to the invention is explained in exemplary embodiments in the drawings, which show respectively:

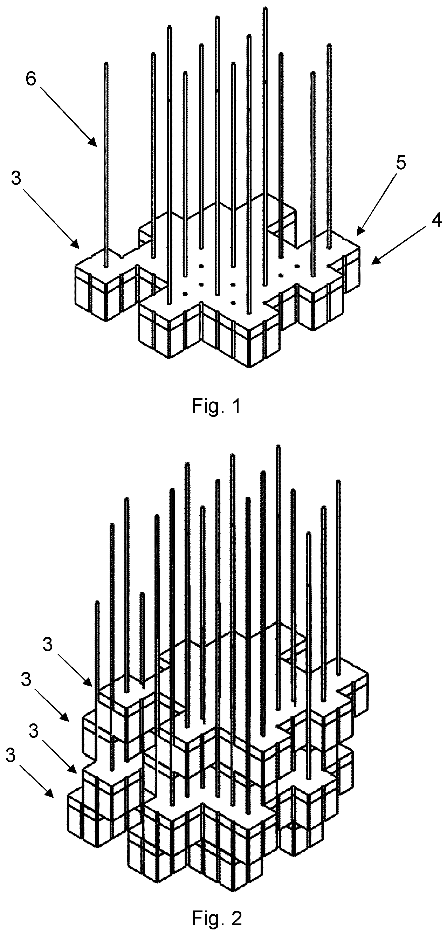

Fig. 1 - a basic cassette with detectors and extended signal-conducting cables in a perspective view, in a version with detectors in the form of cubes,
Fig. 2 - a module of four stacked cassettes, located one above the other, in a perspective view, in a version with detectors in the form of cubes,

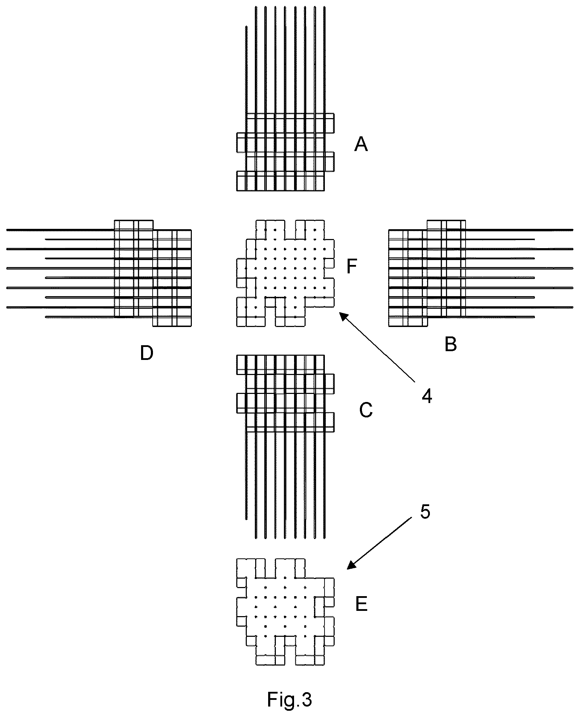

Fig. 3 - a module of four stacked cassettes, located one above the other, in a version with detectors in the form of cubes, in projections:
   A front view
   B left-side view
   C rear view
   D right-side view
   E bottom cover in top view
   F top cover in top view
Fig. 4 - detector covers arranged in the base of the cassette, in a perspective view, in a version with detectors in the form of cubes,
Fig. 5 - detector covers arranged in the cassette cover, in a perspective view, in a version with detectors in the form of cubes,
Fig. 6 - detectors located in covers in the cassette, in cross-section,

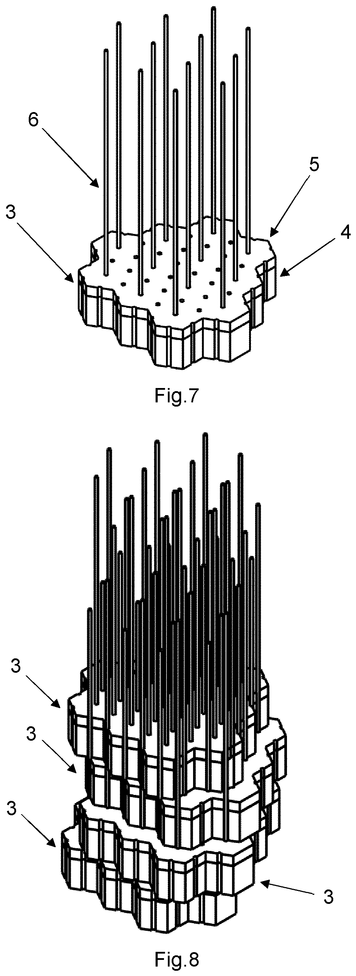

Fig. 7 - a basic cassette with detectors and extended signal-conducting cables in a perspective view, in a version with detectors in the form of regular prisms (honeycomb),
Fig. 8 - a module of four stacked cassettes, located one above the other, in a perspective view, in a version with detectors in the form of regular prisms (honeycomb),

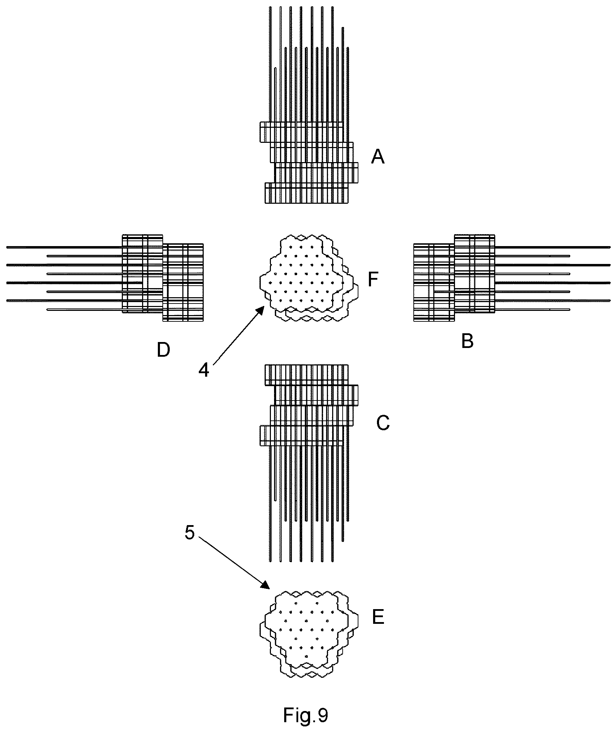

Fig. 9 - a module of four stacked cassettes, located one above the other, in a version with detectors in the form of regular prisms (honeycomb), in projections:
   A front view
   B left-side view
   C rear view
   D right-side view
   E bottom cover in top view
   F top cover in top view
Fig. 10 - detector covers assembled in the base of the cassette, in a perspective view, in a variant with detectors in the form of regular prisms (honeycomb),
Fig. 11 - detector covers assembled in the cassette cover, in a perspective view, in a variant with detectors in in the form of regular prisms (honeycomb),
Fig. 12 - detectors located in covers in the cassette, in cross-section, in a version with detectors in the form of regular prisms (honeycomb),

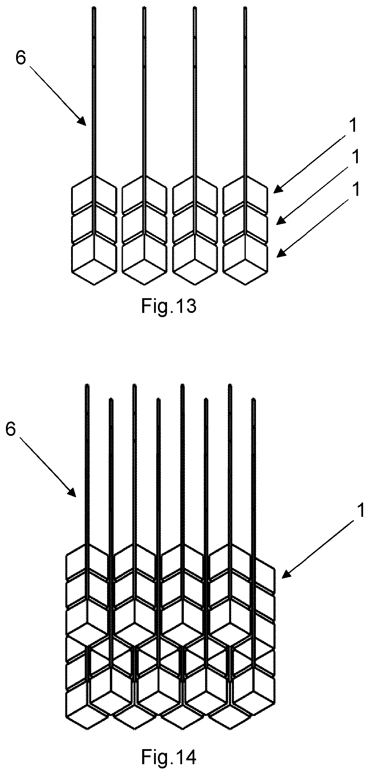

Fig. 13 - basic set with detectors and signal-conducting cables in a perspective view, in a version with detectors in the form of a rhombic dodecahedron,
Fig. 14 - a module of four sets, located one above the other, in a perspective view, in a version with detectors in the form of a rhombic dodecahedron,

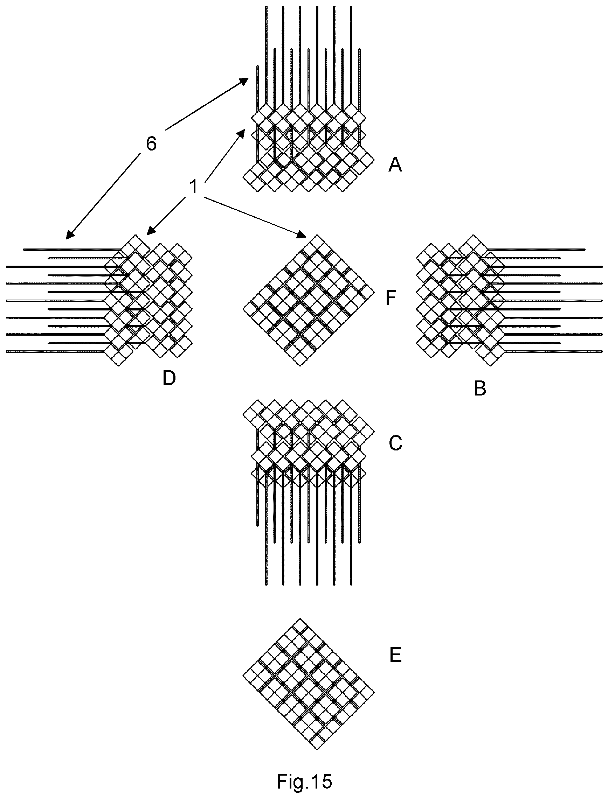

Fig. 15 - a module of four stacked cassettes, located one above the other, in a version with detectors in the form of a rhombic dodecahedron, in projections:
   A front view
   B left-side view
   C rear view
   D right-side view
   E module in bottom view
   F module in top view

In the solution according to the invention, each voxel of the working area of the phantom device corresponds to one individual detector of ionizing radiation.

The basic element of the device is an individual detector 1 in the form of a regular solid shape, preferably in the form of a cube, individually shielded around all faces by shielding 2. The detectors 1 are arranged next to each other in the basic cassettes 3 of the shielding 2, in the discussed embodiment in two layers located one above the other, shifted by a constant step and closed with a lower cover 4 and an upper cover 5 of the shielding 2. Covers 4, 5 on both sides of the cassettes 3 ensure the mechanical rigidity of the structure.

The module of cassettes 3 with detectors 1 in layers is arranged in the active volume in a regular geometric arrangement, fitting the active volume of the phantom device as closely as possible. The optimal shape of the phantom device is a sphere.

A preferred arrangement is 64 individual detectors in 4 cassettes of 16 detectors each, in four layers one above the other.

Detectors 1 in the discussed version are scintillators connected via optical fibers and individual photomultipliers to the signal reading, recording and processing module, not shown in the drawing.

Each of the individual detectors 1 has an independent, individual, straight signal-conducting cable 6. The cable 6 is located in the symmetry axis of the detector 1 along a straight line, not intersecting the volumes of other detectors 1. Shifting the cassettes 3 in the assembled module by a step allows the signal-conducting cables 6 to be moved apart and routed straight-through, without bending and without collisions between the detectors 1 of different layers of the cassettes 3. Straight-line termination and subsequent location without bending, in the case of the signal-conducting cable 6 in the form of an optical fiber, enable proper straight light-guidance, while in the case of such cable 6 being in the form of an electric cable, it minimizes the time delay for the signals from individual detectors to reach the readout and control module.

The covers 5 are equipped with through holes 7 for the signal-conducting cables 6 of the detectors 1 located in the layer of the cassette 3.

The shielding 2 of detectors 1 is equipped in its walls with channels 8 for signal-conducting cables 6 of other detectors located in adjacent layers; in the discussed embodiment with four layers - in the layers below. The channels 8 are located in the walls of the shielding 2, on their common or outer edges. This allows all individual cables 6 of each individual detector 1 to be run independently straightly and in parallel.

Cassettes 3 have an outline defining a puzzle-type fin connector. This shape of the cassette 3, containing one layer of sixteen detectors 1, enables further expansion of the device's volume by coherently connecting subsequent modules with cassettes in the active volume of the phantom device, making the device reconfigurable in terms of hardware.

The second way to reconfigure the device is to programmatically select only specific detectors 1 corresponding to their appropriate voxels. Each detector 1 corresponding to a voxel includes an individual scintillator, optical fiber output and a signal readout system. This makes it possible to assess the dose distribution in a given area and in a given time interval - in the case of analyzing a dynamic procedure (e.g. DCAT or IMAT).

A reconfigurable device whose active area is determined in this way makes it possible to adjust and map the spatial distribution of the dose of ionizing radiation planned to be administered - to the treated volume of the body of an oncology patient undergoing radiotherapy treatment by external beams.

In another embodiment, the regular geometric solid has the form of a regular prism with a regular hexagonal base ("honeycomb"). Due to its shape as a figure closest to a circle, a regular hexagon ensures optimal filling of the active volume of a single layer of the device, taking into account the thickness of the shielding walls of individual detectors.

In another version, it is also possible to shape the basic element in the form of a rhombic dodecahedron as a shape closest to a sphere, ensuring optimal filling of the active volume, taking into account the thickness of the shielding walls of individual detectors. In this case, the space filling is significantly optimized (there are shifts of ½ of the height of each solid in each of the three axes), although it is possible to run straight optical fibers for two layers only, directly adjacent to each other. Rhombic dodecahedral detectors do not require covers.

## Claims

1. A phantom device for teleradiotherapy to determine the difference between the spatial distribution of the delivered dose of ionizing radiation and the distribution of the planned dose over the active volume of the phantom device, containing several individual measurement points corresponding to voxels of the active volume over which the dose distribution is assessed, equipped with individual repeatable ionizing radiation detectors, from each of which signal-conducting cables are led to a module configured to read, record and process the signal, **characterized in that**
i. individual ionizing radiation detectors (1) corresponding to the voxels of the active volume have the form of a sequence of identical regular solids, individually shielded over all faces by shielding (2) and arranged to contact each other, placed within basic cassettes (3), whereby at least two layers of cassettes (3) are located one above the other and are shifted by a regular step, spatially arranged in a repetitive regular geometric manner, fitting the active measuring volume of the phantom device as closely as possible,
ii. each individual detector (1) is connected to an independent, individual signal-conducting cable (6) located along a symmetry axis of this detector, continuing as a straight line and not colliding with other detectors,
iii. the shielding (2) of individual detectors (1) is equipped in its walls with through channels (8) for signal-conducting cables (6) of other detectors located in adjacent layers.

2. The device according to claim 1, wherein the individual detectors (1) are in the form of cubes.

3. The device according to claim 1, wherein the individual detectors (1) have the form of regular prisms with a regular hexagonal base, constituting a honeycomb system.

4. The device according to claim 1, wherein the individual detectors (1) are in the form of rhombic dodecahedra

5. The device according to claim 1, wherein it contains 64 individual detectors (1) in 4 layer modules, 16 in each, shifted by a fixed step.

6. The device according to claim 1, wherein the cassettes (3) are closed with a lower cover (4) and an upper cover (5) of the shielding (2).

7. The device according to claim 1, wherein the cassettes (3) have an outline defining a puzzle-type fin joint.

8. The device according to claim 7, wherein the layered cassette (3) modules are connected to subsequent cassette modules (3) within the active volume of the phantom device.

9. The device according to claim 1, wherein the individual ionizing radiation detectors (1) are scintillators, the shielding (2) is light-tight, and the signal-conducting cables (6) are optical fibers.

10. The device according to claim 5, wherein the dose detectors (1) in the form of scintillators are coupled to individual photomultipliers.

11. The device according to claim 1, wherein the signal-conducting cables (6) led from the individual ionizing radiation detectors (1) are optical fibers or electric cables.

## Patentansprüche

1. Phantomgerät für die Teleradiotherapie zur Bestimmung der Differenz zwischen der räumlichen Verteilung der applizierten Dosis ionisierender Strahlung und der Verteilung der geplanten Dosis über das aktive Volumen des Phantomgeräts, enthaltend mehrere einzelne Messpunkte, die Voxeln des aktiven Volumens entsprechen, über das die Dosisverteilung beurteilt wird, ausgestattet mit einzelnen, wiederholbaren Detektoren für ionisierende Strahlung, von denen jeweils signalleitende Kabel zu einem Modul geführt werden, das zum Lesen, Aufzeichnen und Verarbeiten des Signals konfiguriert ist,
**dadurch gekennzeichnet, dass**
i. die einzelnen Detektoren für ionisierende Strahlung (1), die den Voxeln des aktiven Volumens entsprechen, die Form einer Sequenz identischer, regelmäßiger Körper aufweisen, die allseitig durch eine Abschirmung (2) abgeschirmt und zueinander in Kontakt angeordnet sind, innerhalb von Basiskassetten (3) platziert, wobei mindestens zwei Lagen von Kassetten (3) übereinander angeordnet und um einen regelmäßigen Versatz zueinander verschoben sind, in einer sich wiederholenden, regelmäßigen geometrischen Anordnung räumlich angeordnet, die sich dem aktiven Messvolumen des Phantomgeräts möglichst genau anpasst,
ii. jeder einzelne Detektor (1) mit einem unabhängigen, einzelnen signalleitenden Kabel (6) verbunden ist, das entlang einer Symmetrieachse dieses Detektors verläuft, sich geradlinig fortsetzt und nicht mit anderen Detektoren kollidiert,
iii. die Abschirmung (2) der einzelnen Detektoren (1) in ihren Wänden mit Durchgangskanälen (8) für signalleitende Kabel (6) anderer, in benachbarten Schichten angeordneter Detektoren versehen ist.

2. Gerät nach Anspruch 1, wobei die einzelnen Detektoren (1) die Form von Würfeln aufweisen.

3. Gerät nach Anspruch 1, wobei die einzelnen Detektoren (1) die Form von regelmäßigen Prismen mit regelmäßiger sechseckiger Grundfläche aufweisen und ein wabenförmiges System bilden.

4. Gerät nach Anspruch 1, wobei die einzelnen Detektoren (1) die Form von Rhombendodekaedern aufweisen.

5. Gerät nach Anspruch 1, wobei es 64 einzelne Detektoren (1) in 4 Schichtmodulen mit je 16 Detektoren enthält, die um einen festen Versatz verschoben angeordnet sind.

6. Gerät nach Anspruch 1, wobei die Kassetten (3) mit einer unteren Abdeckung (4) und einer oberen Abdeckung (5) der Abschirmung (2) verschlossen sind.

7. Gerät nach Anspruch 1, wobei die Kassetten (3) eine Kontur aufweisen, die eine puzzleartige Steckverbindung bildet.

8. Gerät nach Anspruch 7, wobei die geschichteten Kassettenmodule (3) innerhalb des aktiven Volumens des Phantomgeräts mit nachfolgenden Kassettenmodulen (3) verbunden sind.

9. Gerät nach Anspruch 1, wobei die einzelnen Detektoren für ionisierende Strahlung (1) Szintillatoren sind, die Abschirmung (2) lichtdicht ist und die signalleitenden Kabel (6) optische Fasern sind.

10. Gerät nach Anspruch 5, wobei die Dosisdetektoren (1) in Form von Szintillatoren mit einzelnen Photomultipliern gekoppelt sind.

11. Gerät nach Anspruch 1, wobei die von den einzelnen Detektoren für ionisierende Strahlung (1) kommenden signalleitenden Kabel (6) optische Fasern oder elektrische Kabel sind.

## Revendications

1. Appareil fantôme pour la téléradiothérapie permettant de déterminer la différence entre la distribution spatiale de la dose de rayonnement ionisant délivrée et la distribution de la dose planifiée sur le volume actif de l'appareil fantôme, comportant plusieurs points de mesure individuels correspondant aux voxels du volume actif sur lesquels la distribution de dose est évaluée, équipé de détecteurs de rayonnement ionisant individuels et reproductibles, dont chacun est relié par un câble conducteur de signal à un module configuré pour lire, enregistrer et traiter le signal,
**caractérisé en ce que**
i. les détecteurs de rayonnement ionisant individuels (1) correspondant aux voxels du volume actif se présentent sous la forme d'une séquence de solides réguliers identiques, blindés individuellement sur toutes leurs faces par un blindage (2) et disposés en contact les uns avec les autres, placés dans des cassettes de base (3), au moins deux couches de cassettes (3) étant superposées et décalées d'un pas régulier, disposées spatialement de manière géométrique régulière et répétitive, épousant au mieux le volume de mesure actif de l'appareil fantôme,
ii. chaque détecteur individuel (1) est relié à un câble conducteur de signal indépendant (6), situé le long de son axe de symétrie, se prolongeant en ligne droite et n'entrant pas en collision avec d'autres détecteurs,
iii. le blindage (2) des détecteurs individuels (1) est muni, dans ses parois, de canaux traversants (8) pour les câbles conducteurs de signal (6) d'autres détecteurs situés dans les couches adjacentes.

2. Appareil selon la revendication 1, dans lequel les détecteurs individuels (1) se présentent sous la forme de cubes.

3. Appareil selon la revendication 1, dans lequel les détecteurs individuels (1) se présentent sous la forme de prismes réguliers à base hexagonale régulière, constituant une structure en nid d'abeille.

4. Appareil selon la revendication 1, dans lequel les détecteurs individuels (1) se présentent sous la forme de dodécaèdres rhombiques.

5. Appareil selon la revendication 1, dans lequel il comprend 64 détecteurs individuels (1) en 4 modules de couche, à 16 détecteurs chacun, décalés d'un pas fixe.

6. Appareil selon la revendication 1, dans lequel les cassettes (3) sont fermées par un couvercle inférieur (4) et un couvercle supérieur (5) du blindage (2).

7. Appareil selon la revendication 1, dans lequel les cassettes (3) présentent un contour définissant un assemblage à emboîtement de type puzzle.

8. Appareil selon la revendication 7, dans lequel les modules de cassettes (3) superposés sont connectés aux modules de cassettes (3) successifs au sein du volume actif de l'appareil fantôme.

9. Appareil selon la revendication 1, dans lequel les détecteurs de rayonnements ionisants (1) sont des scintillateurs, le blindage (2) est étanche à la lumière et les câbles conducteurs de signal (6) sont des fibres optiques.

10. Appareil selon la revendication 5, dans lequel les détecteurs de dose (1), sous forme de scintillateurs, sont couplés à des photomultiplicateurs individuels.

11. Appareil selon la revendication 1, dans lequel les câbles conducteurs de signal (6) provenant des détecteurs de rayonnement ionisant individuels (1) sont des fibres optiques ou des câbles électriques.
